Europäisches Patentamt

European Patent Office

Office européen des brevets

(11) Publication number: **0 371 752**
**A1**

## EUROPEAN PATENT APPLICATION

(21) Application number: **89312343.0**

(22) Date of filing: **28.11.89**

(51) Int. Cl.5: **A61M 16/04, A61M 25/01**

(30) Priority: **01.12.88 GB 8828012**

(43) Date of publication of application:
**06.06.90 Bulletin 90/23**

(84) Designated Contracting States:
**DE FR GB IT SE**

(71) Applicant: **Shetty, Devi Cardiothoracic Unit
B.M. Birla Heart Centre 7/2 Diamond Harbour
Road
Calcutta 700027(IN)**

(72) Inventor: **Shetty, Devi Cardiothoracic Unit
B.M. Birla Heart Centre 7/2 Diamond Harbour
Road
Calcutta 700027(IN)**

(74) Representative: **Flint, Jonathan McNeill
SMITHS INDUSTRIES PUBLIC LIMITED
COMPANY 765 Finchley Road
London NW11 8DS(GB)**

(54) Tracheostomy tube assemblies.

(57) A tracheostomy tube obturator (2) has a nose (20) of silicone rubber which projects from the patient end (10) by a distance about two and a half times the internal diameter of the trachestomy tube (1). The nose (20) is circular in section, tapering to its tip (21), and is bent along its length, in the same plane and sense as the tube (1) so as to aid introduction of the tube into a stoma (4). The stem (22) of the obturator (2) is flexible and cruciform in section and is of a different material from that of he nose (20). At its machine end, the obturator (2) has a flange (23) with a projection (24) on one side which is aligned with a marking or recess on the machine end of the tube (1) so as to ensure correct orientation of the obturator in the tube.

Fig. 1.

# TRACHEOSTOMY TUBE ASSEMBLIES

This invention relates to tracheostomy tube assemblies of the kind comprising a tracheostomy tube with a patient end adapted for location within the trachea of a patient and a machine end adapted to extend through a surgically made opening into the trachea and to be located externally of the trachea, and an obturator for use in insertion of the tracheostomy tube.

Tracheostomy tubes are used to provide an airway or gas ventilation path directly to the patient's trachea through a surgically made opening in the throat. In order to cause minimal trauma to the patient, the opening into the trachea is preferably made just large enough to accommodate the tube. This can make insertion of the tube through the opening difficult, since it must be pushed through resilient cartilage. Obturators can be used to provide the tube with additional stiffness and to prevent entry of tissue into the patient end of the tube. Such obturators do not significantly help insertion since they only project from the patient end by a maximum distance about equal to the internal diameter of the tube.

It is an object of the present invention to provide an improved tracheostomy tube assembly.

According to the present invention there is provided a tracheostomy tube assembly of the above-specified kind, characterised in that the obturator extends along the tracheostomy tube and projects from the patient end thereof, that the obturator includes a flexible stem and a nose portion at the patient end of the obturator, the nose portion being of a soft resilient material, and tapering to its tip, that the nose portion is arranged to project out of the patient end of the tube by a distance at least twice the internal diameter of the tube at its patient end such that the nose portion forms a lead for introduction of the assembly into the trachea, and that the stem and the nose portion are more flexible than the tube such that the obturator means can be withdrawn from the tube after insertion by pulling the machine end of the obturator without any substantial bending of the tube.

The nose portion preferably projects from the patient end of the tube by a distance approximately two and a half times the internal diameter of the tube at its patient end. The nose portion is preferably curved along its length and may have a Shore hardness of approximately 83. The nose portion may be of silicone rubber and may be of substantially circular section. The stem is preferably of a material different from that of the nose portion and may be cruciform in section along a major part of its length. The patient end of the obturator may be treated to be of low friction. Preferably, the machine end of the obturator and the tube both have respective means which are aligned with one another to ensure correct orientation of the obturator in the tube.

A tracheostomy tube assembly including an obturator, and a method of inserting a tracheostomy tube, in accordance with the present invention, will now be described, by way of example, with reference to the accompanying drawings, in which:

Figure 1 is a side elevation showing the assembly inserted in a trachea;

Figure 2 is a side elevation view of the obturator;

Figure 3 is an enlarged transverse section of the obturator along line III-III; and

Figure 4 is a side elevation showing a step in the insertion of the assembly.

The tracheostomy tube assembly comprises a conventional tracheostomy tube 1 and a novel obturator 2 used for introducing the tube into the patient's trachea.

The tracheostomy tube 1 is of a semi-flexible plastics material such as PVC, having a patient end 10 that is, in use, located in the trachea 3. The patient end of the tube is straight and carries an inflatable cuff 11 that encircles it and which forms a seal with the trachea when inflated.

The machine end 12 of the tube is also straight and extends at right angles to the patient end 10, being joined with it by an integral intermediate curved region 13. The machine end 12 extends through a surgically cut opening or stoma 4 and is located outside the trachea, close to the patient's neck, being terminated by a luer taper male coupling 14. A flange 15 is also mounted on the tube to serve in stabilizing the tube at the stoma. An inflation line 16 communicates with the interior of the cuff 11 via a bore (not shown) along the tube 1 within its wall. The inflation line 16 is joined to the bore close to the machine end 12 and includes an inflation indicator 17 and coupling 18 of the usual kind.

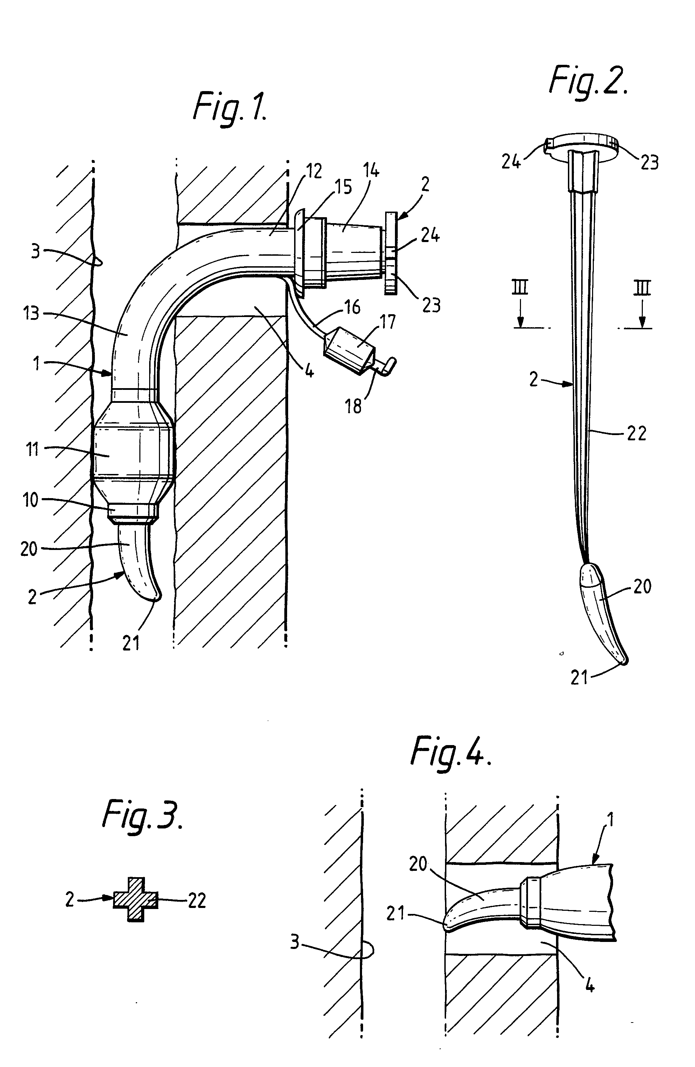

With reference now to Figures 2 and 3, the obturator 2 has, at its patient end, a nose portion 20 of a soft, resilient, elastomeric material such as silicone rubber. Typically the hardness of the nose portion is about 83 Shore hardness. The nose portion 20 is of circular section and tapers along its length to a rounded tip 21. The length of the obturator 2 is such that the nose portion 20 projects from the patient end 10 of the tube 1 by a distance that is at least twice the internal diameter of the patient end of the tube and is preferably two and half times the diameter. The nose portion 20 is curved along its length slightly to the right in the drawings, that is, towards the front surface of the patient's neck, and in the same plane and sense as the bend in the tube 1. The diameter of the nose portion 20 where it emerges from the patient end of the tube is the same as the internal diameter of the tube so that it is a sliding fit in the tube and seals its patient end. The nose portion 20 is securely attached to the patient end of a stem portion 22 which is of a semi-flexible plastics material, such as nylon. The stem portion 22 is of cruciform section along a major part of its length, and tapers, along its length, being smaller at its patient end. At its machine end, the stem portion is formed with a radially-extending stop 23 of disc shape. A projection 24 from the edge of the stop 23 serves to identify the orientation of the obturator. The cruciform section of the obturator gives it sufficient axial rigidity to enable it to be inserted into the tube against the action of friction between the nose portion 20 and the tube wall. The friction can be reduced by lubricating the forward end of the obturator or otherwise treating it to be of low friction such as by a suitable coating. The dimensions and material of the stem 22 ensure that it is relatively flexible transversely, that is, more flexible than the tube 1.

In use, the obturator 2 is pushed into the tube 1 as far as possible, the extent of insertion being limited by engagement of the stop 23 with the coupling 14. The projection 24 is aligned with a marking, or a cooperating recess (not shown), on the tube 1 to ensure that the nose portion 20 bends in the desired direction. A lock may also be provided to hold the obturator in position.

With the assembly completed, the tip 21 of the nose portion is pushed into the surgically made stoma 4, in the manner shown in Figure 4. The length of the obturator 2 projecting from the tube 1 ensures that the region of the stoma 4 can be clearly seen by the surgeon and is not obstructed by the tube during the initial part of the insertion. As the assembly is pushed into the stoma 4, the stoma is opened progressively by the tapering tip 21 of the obturator 2 which guides the assembly through the stoma. As the assembly enters the trachea 3, the surgeon bends the assembly downwards to follow the lead of the obturator tip. If the assembly is pushed too far to the rear before being bent down, the tip 21 of the obturator 2 may contact the rear surface of the wall of the trachea. The soft nature of the nose portion 20, however, ensures that this causes little risk of trauma, and the bend of the obturator ensures that the patient end of the assembly is guided downwardly into its correct position shown in Figure 1.

When the assembly has been correctly positioned, with the flange 15 close to the patient's neck, the obturator 20 is quickly removed by pulling the stop 23 rearwardly. The flexible nature of the stem portion 22 and nose portion 20 ensures that the obturator can bend to follow the changes of curvature of the tube 1 along its length without causing any substantial bending of the tube.

Once the obturator has been removed, the cuff 11 can be inflated to seal the tube with the trachea 3.

The obturator 20 has been found greatly to assist correct intubation and to reduce the discomfort to the patient.

It will be appreciated that the obturator can be used with tubes of different sizes as commonly used for patients of different builds. The obturator can be of different shapes and materials whilst still being within the scope of the present invention.

## Claims

1. A tracheostomy tube assembly comprising a tracheostomy tube with a patient end adapted for location within the trachea of a patient and a machine end adapted to extend through a surgically made opening into the trachea and to be located externally of the trachea, and an obturator for use in insertion of the tracheostomy tube, characterised in that the obturator (2) extends along the tracheostomy tube (1) and projects from the patient end (10) thereof, that the obturator includes a flexible stem (22) and a nose portion (20) at the patient end of the obturator, the nose portion (20) being of a soft resilient material and tapering to its tip (21), that the nose portion (20) projects out of the patient end (10) of the tube (1) by a distance at least twice the internal diameter of the tube at its patient end such that the nose portion (20) forms a lead for introduction of the assembly into the trachea (3), and that the stem (22) and the nose portion (20) are more flexible than the tube such that the obturator can be withdrawn from the tube (11) after insertion by pulling the machine end of the obturator without any substantial bending of the tube.

2. A tracheostomy tube assembly according to Claim 1, characterised in that the nose portion (20) projects from the patient end (10) of the tube (1) by a distance approximately two and a half times the internal diameter of the tube (1) at its patient end (10).

3. A tracheostomy tube assembly according to Claim 1 or 2, characterised in that the nose portion (20) is curved along its length.

4. A tracheostomy tube assembly according to any one of the preceding claims, characterised in that the nose portion (20) has a Shore hardness of approximately 83.

5. A tracheostomy tube assembly according to any one of the preceding claims, characterised in that the nose portion (20) is substantially of a silicone rubber.

6. A tracheostomy tube assembly according to any one of the preceding claims, characterised in that nose portion (20) is of a substantially circular section.

7. A tracheostomy tube assembly according to any one of the preceding claims, characterised in that the stem (22) is of a material different from that of the nose portion (20).

8. A tracheostomy tube assembly according to any one of the preceding claims, characterised in that the stem (22) is cruciform in section along a major part of its length.

9. A tracheostomy tube assembly according to any one of the preceding claims, characterised in that the patient end of the obturator (2) is treated to be of low friction.

10. A tracheostomy tube assembly according to any one of the preceding claims, characterised in that the machine end of the obturator (2) and tube (1) both have respective means (24) which are aligned with one another to ensure correct orientation of the obturator (2) in the tube (1).

Fig.1.

Fig.2.

Fig.3.

Fig.4.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 246 897 (MUTO)<br>* Column 2, line 60 - column 3, line 46; figures 1,3 * | 1,2,6 | A 61 M 16/04<br>A 61 M 25/01 |
| Y | US-A-3 606 669 (KEMBLE)<br>* Column 2, lines 55-69; column 4, lines 9-19; figure 5 * | 1,2,4-6,10 | |
| Y | EP-A-0 214 063 (BAZENET)<br>* Column 3, lines 7-31; figures 1,9 * | 1,2,4-6,10 | |
| Y | US-A-1 920 006 (DOZIER)<br>* Page 2, lines 35-44; figure 3 * | 10 | |
| X | US-A-4 502 482 (DE LUCCIA)<br>* Column 4, lines 2-49; column 5, line 68 - column 6, line 21; figures 1-4 * | 1-3,6,7 | |
| A | US-A-4 637 388 (MELEDY)<br>* Column 3, lines 16-28; column 3, lines 41-45 * | 7 | TECHNICAL FIELDS SEARCHED (Int. Cl.5) |
| A | DE-A-3 530 310 (STERIMED)<br>* Page 4, lines 14-22 * | 8 | A 61 M |
| A | FR-A-2 247 262 (TECHNOLOGICAL SUPPLY)<br>* Page 1, lines 10-38 * | 9 | |

The present search report has been drawn up for all claims

| Place of search | Date of completion of the search | Examiner |
|---|---|---|
| THE HAGUE | 26-02-1990 | SCHOENLEBEN J.E.F. |